(19) Europäisches Patentamt
European Patent Office
Office européen des brevets

(11) **EP 4 400 828 A1**

(12) **EUROPEAN PATENT APPLICATION**
published in accordance with Art. 153(4) EPC

(43) Date of publication:
**17.07.2024 Bulletin 2024/29**

(21) Application number: **22949674.0**

(22) Date of filing: **16.11.2022**

(51) International Patent Classification (IPC):
**G01N 21/25** (2006.01)   **G06N 20/00** (2019.01)

(52) Cooperative Patent Classification (CPC):
G01N 2201/129; G01N 2201/1296

(86) International application number:
**PCT/KR2022/018141**

(87) International publication number:
**WO 2024/106564 (23.05.2024 Gazette 2024/21)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **LG Chem, Ltd.**
**Seoul 07336 (KR)**

(72) Inventors:
• **KIM, Jae Hyun**
  **Daejeon 34122 (KR)**

• **KIM, Byung Mook**
  **Daejeon 34122 (KR)**
• **LEE, Jin Yeong**
  **Daejeon 34122 (KR)**
• **LEE, Kyu Hwang**
  **Daejeon 34122 (KR)**
• **SONG, Young Soo**
  **Daejeon 34122 (KR)**

(74) Representative: **Hoffmann Eitle**
**Patent- und Rechtsanwälte PartmbB**
**Arabellastraße 30**
**81925 München (DE)**

(54) **SPECTRAL LEARNING-BASED APPARATUS AND METHOD FOR MEASURING CONCENTRATION OF SUBSTANCE**

(57)   A method for generating a concentration prediction model for predicting a concentration of a target substance through machine learning. The method includes a training data generation step of generating an optimal transformed spectrum obtained by transforming a basic spectrum of a substance of a known concentration according to a predetermined transformation condition as training data, and a concentration prediction model generation step of generating a concentration prediction model by machine learning the optimal transformed spectrum generated in the training data generation step and transformed according to the predetermined transformation condition and an actually measured concentration of a substance corresponding to the optimal transformed spectrum, and the method may improve accuracy in substance concentration prediction by suppressing a spectral change caused by compounds other than an analyte to be predicted and maximizing the spectral change with a concentration of the analyte.

Fig. 1

**Description**

## TECHNICAL FIELD

The present invention relates to a method for checking the concentration of a substance in a solution in real time, and more particularly, to a method for accurately predicting the concentration of a substance to be analyzed by learning optical analysis spectra of a process solution with a varying substance concentration in real time based on a concentration prediction algorithm.

## BACKGROUND ART

In order to acquire chemical information about a substance in a solution, spectra may be acquired through optical analysis methods using spectrometers such as IR, Raman, and UV-vis, and the chemical information about the substance is extracted based on the acquired spectra, and the concentration thereof is calculated.

For the substance concentration analysis through the optical analysis method in the related art, it is difficult to distinguish chemical information about substances based on components due to the mixed state of various chemicals, and it takes a lot of time to distinguish and analyze them, and as a consequence, a problem with difficulty in measuring the concentration of the analyte arises in real time. In order to solve the problem, the related art predicts the analysis of the analyte through multivariate linear combinations by using multi-regression analysis, polynomial regression, partial least squares (PLS) or net analyte signal (NAS) algorithms based on machine learning. The methods are presented in Patent Document 1. However, when the relationship between the concentration of the analyte and the spectrum obtained from a spectrometer is not linear, a problem arises in that the accuracy of algorithm model formation and concentration prediction is low. In addition, there is a problem that the learning rate of the model drops when a change in the spectrum with the concentration of an analyte in a solution is insignificant.

Meanwhile, using a decision tree, a random forest, a support vector machine, deep-learning, or the like, which are able to perform nonlinear regression learning based on machine learning, the concentrations may be predicted from the spectrum of an analyte. However, the methods generate a prediction model only by adjusting learning parameters for the spectrum, which may result in lowering of the accuracy of predicting the concentration of a target substance due to various substance information included in the spectrum.

In order to improve the above-mentioned problem, Patent Document 2 attempts more accurate concentration prediction through spectrum transformation through comparison of background signals and similarity intervals.

However, the related arts predict the concentration through simple transformation of the spectrum, and thus, do not provide a sufficient spectral preprocessing technology for optimal concentration prediction. In order to solve the above-described problems, the present invention is to provide an analyte concentration prediction method and system capable of improving the accuracy of a concentration prediction model by performing machine learning with a transformed spectrum of a substance to generate the concentration prediction model and transforming a spectrum of an analyte and inputting the transformed spectrum into the generated concentration prediction model.

Related arts include the following patent documents.

(Patent Document 1) KR-2020-0018177 A
(Patent Document 2) JP 6871195 B2

## DISCLOSURE OF THE

## TECHNICAL PROBLEM

The present invention has been made to solve the above-described problems, and is to provide an analyte concentration prediction method capable of improving the accuracy of concentration prediction by adding a spectrum transformation algorithm of an analyte to a concentration prediction model.

## TECHNICAL SOLUTION

There is provided a method for generating a concentration prediction model for predicting a concentration of a target substance through machine learning, the method including: generating a transformed spectrum by transforming a base spectrum of a substance of a known concentration according to a predetermined transformation condition; and generating a concentration prediction model by inputting the transformed spectrum and a measured concentration of a substance corresponding to the transformed spectrum into a machine learning algorithm.

The predetermined transformation condition may be derived from: transformed spectra generated by transforming a spectrum with two or more different transformation conditions.

Deriving the optimal transformation condition may include calculating each of a reference similarity standard deviation of the transformed spectra, a correlation coefficient with the measured concentration, and a multivariate ratio before and after spectrum transformation, and the transformation condition may be a sum of the reference similarity standard deviation, the correlation coefficient and the multivariate ratio is maximum.

There is provided a non-transitory computer-readable medium having a concentration prediction model generation algorithm programmed thereon, the concentration prediction model generation algorithm including: a spectrum transformation module for generating transformed spectra corresponding to base spectra by transforming the base spectra according to two or more transformation conditions; a transformed spectrum calculation module for calculating each of a reference similarity standard deviation of each of the transformed spectra according to the transformation conditions, a correlation coefficient with a measured concentration of a substance corresponding to each of the transformed spectra, and a multivariate ratio before and after spectrum transformation, and calculating the transformation spectra according to a transformation condition for which a sum of the reference similarity standard deviation, the correlation coefficient and the multivariate ratio is maximum; and a machine learning algorithm generation module for generating a concentration prediction model using measured concentrations of substances corresponding to the transformed spectra as training data.

There is provided a concentration prediction method for predicting a concentration of an analyte, the concentration prediction method including: acquiring a prediction spectrum of a substance to be predicted in concentration; generating a transformation prediction spectrum by transforming the acquired prediction spectrum under a transformation condition; and outputting a concentration prediction value by inputting the transformation prediction spectrum to a concentration prediction model, in which the optimal transformation condition is a transformation condition derived from generating transformed spectra by transforming base spectra of predetermined substances having concentration values known by measurement with two or more different transformation conditions; and deriving the transformation condition from the generated transformed spectra, and the concentration prediction model is a neural network or a concentration prediction algorithm trained to calculate a predicted concentration value from transformed spectra obtained by transforming the base spectra with the transformation condition by inputting the transformed spectra and measured concentration values corresponding to the transformed spectra into a machine learning algorithm.

Deriving the optimal transformation condition may include calculating each of a reference similarity standard deviation of the transformed spectra, a correlation coefficient with the measured concentration, and a multivariate ratio before and after spectrum transformation, and the transformation condition may be a sum of the reference similarity standard deviation, the correlation coefficient and the multivariate ratio is maximum.

There is provided a concentration prediction system for predicting a concentration of an analyte, the concentration prediction system including: a spectrum data acquisition unit configured to acquire a base spectrum of a substance for generating training data or a prediction spectrum that is a spectrum of a substance to be measured in concentration; a substance concentration data acquisition unit configured to acquire a measured concentration of the substance to be measured through previously acquired concentration data for the substance and a concentration measuring device; a calculation and control device configured to generate transformed spectra of the base spectrum and the prediction spectrum, calculate a transformation condition, perform machine learning, and generate a concentration prediction model; and a memory for storing the base spectra of analytes acquired by the spectrum data acquisition unit, measured substance concentration values acquired by the substance concentration data acquisition unit, and the concentration prediction model generated by the calculation and control device.

The calculation and control device may be configured to perform a spectrum transformation and combination of spectra acquired in the spectrum data acquisition unit according to a spectrum transformation algorithm, derive a transformed spectrum exhibiting concentration prediction accuracy as a transformed spectrum, among the transformed spectra, among the transformed spectra, generate a concentration prediction model by performing machine learning using the transformed spectrum and the measured concentration of a corresponding substance as training data, and perform concentration prediction by inputting the prediction spectrum into the generated concentration prediction model.

The memory may include: a spectrum transformation and combination module for transforming the spectrum acquired by the spectrum acquisition unit according to the transformation condition; a spectrum derivation module for acquiring a spectrum exhibiting concentration prediction accuracy among the transformed spectra and deriving the transformation condition; a machine learning module for performing machine learning and generating a concentration prediction model using the transformed spectrum and measured substance concentration as training data; and a concentration prediction module for calculating a predicted concentration value by using the prediction spectrum as input data.

There is provided a non-transitory computer-readable medium having a concentration prediction algorithm programmed thereon, the concentration prediction algorithm including: a spectrum transformation module for generating a transformed spectrum by transforming a prediction spectrum of a substance to be predicted in concentration; a spectrum derivation module for calculating a transformation prediction spectrum by calling the spectrum transformation module and transforming the prediction spectrum according to a transformation condition; and a concentration prediction module for receiving the transformation prediction spectrum and calculating a predicted concentration value, in which the concentration prediction module is generated by performing machine learning based on a measured concentration of a substance corresponding to the spectrum transformed according to the transformation condition.

There is provided a concentration measuring device for predicting a concentration of an analyte, the concentration measuring device including: a spectrum data acquisition unit configured to acquire a prediction spectrum that is a spectrum of an analyte; a memory device having programmed thereon a spectrum transformation module for transforming a prediction spectrum according to a transformation condition and a concentration prediction module for calculating a concentration prediction value from a transformation prediction spectrum obtained by transforming the prediction spectrum according to the transformation condition; and a calculation and control device configured to perform a control to read the concentration prediction module loaded in the memory device and calculate a concentration prediction value from a prediction spectrum to be predicted in concentration.

The concentration measuring device may further include a data connection unit configured to connect an external memory device or receive data from the external memory device, and the memory device may be connected to the data connection unit in a detachable manner.

The concentration measuring device may further include a data connection unit configured to connect an external memory device or receive data from the external memory device, and the memory device may be configured to receive and store a spectrum transformation module and a concentration prediction module from the external memory device through the data connection unit, wherein the spectrum transformation module is further for transforming the prediction spectrum according to a transformation condition,. The data connection unit may be integrated with the spectrum data acquisition unit.

## ADVANTAGEOUS EFFECTS

The present invention may generate a concentration prediction model by performing machine learning with a transformed spectrum of a substance, and transform the spectrum of the analyte and input the transformed spectrum to the generated concentration prediction model, thereby making it possible to improve the accuracy of the concentration prediction model.

Furthermore, in the transformation of the spectrum, the present invention may improve the accuracy in predicting the concentration prediction of a substance by deriving the optimal transformation method (transformation condition) among various transformation methods, generating a concentration prediction model with the transformed spectrum transformed with the derived optimal transformation method (transformation condition), and using the generated concentration prediction model to predict the concentration.

## BRIEF DESCRIPTION OF THE DRAWINGS

FIG. 1 is a diagram showing a concentration prediction system and device for predicting the concentration of an analyte of the present invention.

FIG. 2 is a block diagram of a concentration measuring device according to the present invention.

FIG. 3 is a diagram of a spectrum transformation method using an inverse transformation method according to an embodiment of the present invention.

FIG. 4 is a diagram of a spectrum transformation method using a filtering transformation method according to an embodiment of the present invention.

FIG. 5 is a diagram of a spectrum transformation method using a baseline removal transformation method according to an embodiment of the present invention.

FIG. 6 is diagram of a spectrum transformation method using an interval averaging transformation method according to an embodiment of the present invention.

FIG. 7 is a diagram of a spectrum transformation method using an interval removal transformation method according to an embodiment of the present invention.

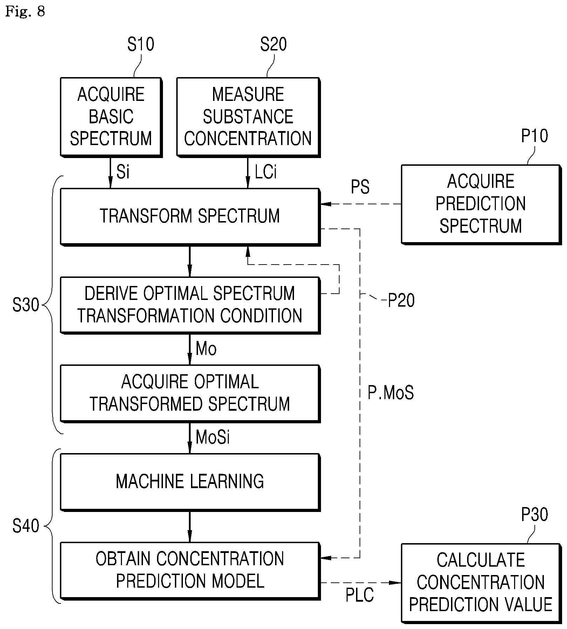

FIG. 8 is a flowchart showing a concentration prediction method for generating a concentration prediction model of the present invention and predicting the concentration of an analyte.

FIG. 9 is a graph showing the mean squared error according to an optimal spectrum transformation condition of the present invention.

FIG. 10 is a graph showing a concentration prediction result of the concentration prediction method in the related art.

FIG. 11 is a graph showing a concentration prediction result of the concentration prediction method in the present invention.

## MODE FOR CARRYING OUT THE INVENTION

Hereinafter, exemplary embodiments of the present disclosure will be described in detail with reference to the accompanying drawings so that those skilled in the art may easily perform the present disclosure. However, the present disclosure may be implemented by various modifications and is not limited to the exemplary embodiments described herein. In the drawings, in order to clarify the present disclosure, parts that are not related to description are omitted and like reference numerals represent like elements throughout the specification.

Hereinafter, exemplary embodiments will be described in detail with reference to the drawings.

The present invention relates to an analyte concentration prediction method for predicting the concentration of an analyte.

More specifically, the present invention relates to a substance concentration prediction system and method with improved accuracy by adding a spectrum transformation algorithm to a substance concentration prediction model.

### 1. Concentration prediction model generation and concentration prediction method of present invention

A concentration prediction model of the present invention is generated by machine learning to predict a concentration value by transforming a spectrum of a substance of a known concentration, which is acquired from a device capable of acquiring spectra of substances, such as a known spectrometer, or of a substance for which a concentration is to be measured experimentally and learning the transformed spectrum.

### 1.1 Training data generation step

In the present invention, a transformed spectrum obtained by transforming a spectrum of a substance of a known concentration as described above and an actually measured concentration of the substance are used as training data. In addition, in order to generate an optimal prediction model, an optimal spectrum transformation condition for generating an optimal prediction model is obtained among spectrum transformation conditions by various spectrum transformation methods and combinations thereof, and transformed spectrum data transformed under the optimal spectrum transformation condition and the actually measured concentration of the corresponding substance are used as training data.

### 1.1.1 Acquisition step for basic spectrum (Si) data (S10)

In the present invention, i basic spectra to be used as training data are expressed as Si. In the present invention, the basic spectrum refers to first spectrum data acquired using a known spectrometer or the like. In the present invention, the acquired i spectra S are expressed as $S_1, S_2, ..., S_i$.

### 1.1.2 Acquisition step for actually measured substance concentration data (LCi) (S20)

Actually measured concentration data LCi for the substances corresponding to each of the above-described basic spectra is acquired. The concentration data LCi for each substance uses known substance concentration data when the basic spectrum Si is acquired for a substance of a concentration that is already known, and may be a value obtained by experimentally measuring the concentration of a substance for which the basic spectrum is acquired through a known concentration measuring device linked to a concentration measuring system of the present invention.

### 1.1.3 Acquisition Step for optimal transformed spectrum (MoSi) (S30)

The transformed spectrum used as training data in the present invention is a spectrum obtained by transforming the above-described basic spectrum according to transformation conditions to be described later.

In the present invention, for description, the spectrum transformation conditions may be set to k types of transformation conditions according to the transformation conditions to be described later and combinations thereof, first, second, ..., k-th transformation conditions are expressed as M1, M2, ..., Mk, respectively, and the optimal transformation condition for generating the optimal concentration prediction model is expressed as Mo.

It will be apparent to those skilled in the art that transformed spectra transformed under each transformation condition may be expressed as M1S1, M1S2, ..., M1Si, for example, when basic spectra (Si, i = 1, 2, ..., n) are transformed under a transformation condition M1, and the transformed spectra may be expressed as MkSi (k = 1, 2, ..., n, i = 1, 2, ..., m) when the basic spectra are transformed under a transformation condition Mk.

In the present invention, among the transformed spectra MkSi, the transformed spectrum MoSi transformed under Mo, which is the optimal transformation condition exhibiting the optimal concentration prediction accuracy, is used as training data.

**(1) Spectrum transformation and combination step**

This is a step of transforming and combining the basic spectra Si according to various transformation conditions in order to derive the optimal transformation condition.

Below, examples of five types of basic spectrum transformation conditions used in the present invention are shown. In the present invention, one of the following five types of transformation methods may be used or at least two of them may be selectively combined to be used as spectrum transformation conditions; however, the present invention is not limited to the five types of methods and combinations thereof, and spectrum transformation conditions known prior to the filing date of the present invention and combinations thereof may be used as the spectrum transformation conditions.

① Transformation condition 1: Inverse transformation

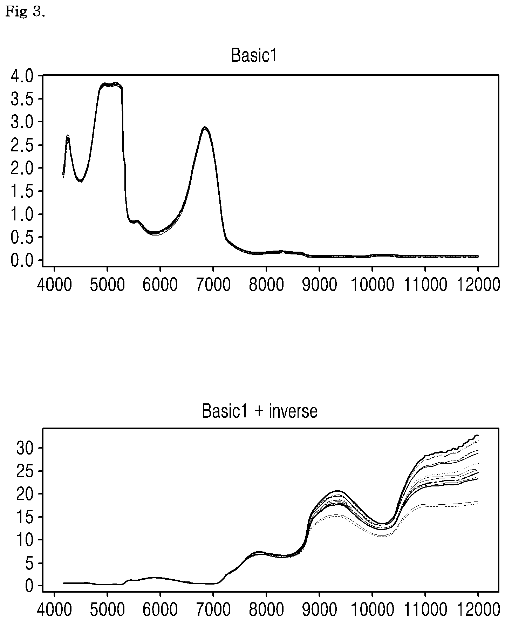

FIG. 3 shows a spectrum transformation method using an inverse transformation method according to an embodiment of the present invention. Inverse transformation refers to transformation that reciprocates data values of the acquired spectrum. For example, when the intensity of a spectrum is assumed to be $I_x$, the intensity of a spectrum appearing through inverse transformation may be transformed into $\dfrac{1}{I+R}$ . In this case, R is a variable that eliminates spectra having large deviations, and prevents the acquired inverse data for transformed spectra from showing deviations of a certain level or greater.

② Transformation Condition 2: Filtering Transformation

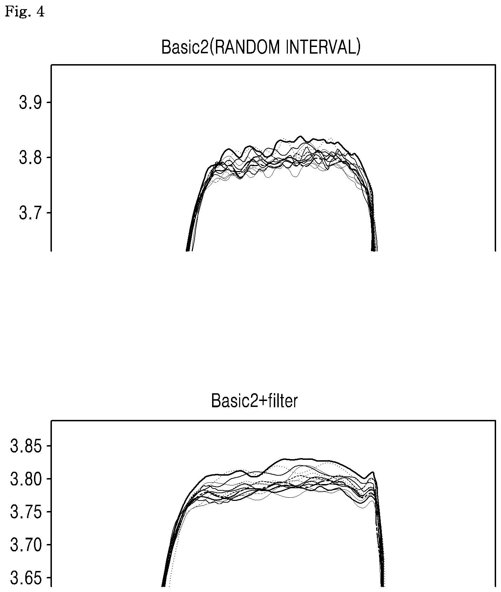

FIG. 4 shows a spectrum transformation method using a filtering transformation method according to an embodiment of the present invention. Filtering transformation refers to performing transformation in a method of selectively blocking a part of an acquired spectrum while allowing the rest to travel through. A relationship between an actual analyte and an acquired spectrum may be improved by filtering out peaks irrelevant to the analyte among spectrum peaks.

③ Transformation Condition 3: Baseline Removal Transformation

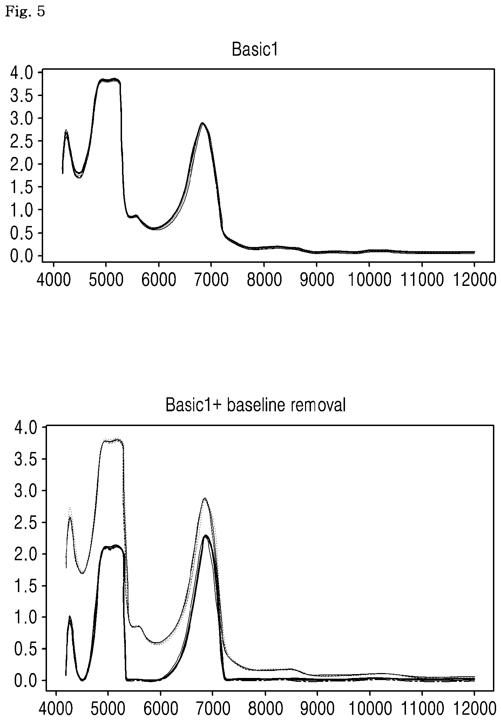

FIG. 5 shows a spectrum transformation method using a baseline removal transformation method according to an embodiment of the present invention. When a spectrum is acquired using a spectrometer, data for the spectrum does not have an ideal baseline due to noise caused by the characteristics of a system, non-uniformity of a sample of a substance to be measured, or the like. Accordingly, the relationship between the concentration of the substance to be measured and the spectrum may be improved by removing the baseline in order to remove the influence of the movement or the like of the baseline in the data for the spectrum. In the case of baseline removal transformation, similarity of data between spectra may increase, and as a result, whether to use the baseline removal transformation may be determined according to the type of substance to be measured or analysis method.

④ Transformation Condition 4: Interval Averaging Transformation

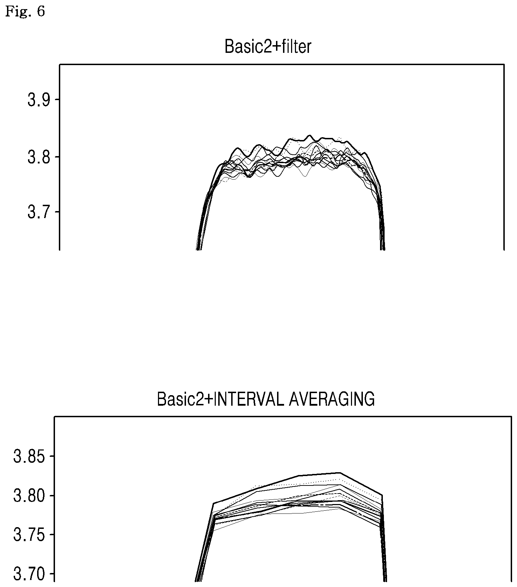

FIG. 6 shows a spectrum transformation method using an interval averaging transformation method according to an embodiment of the present invention. Interval averaging transformation refers to a transformation in which data values of a predetermined interval are averaged in the acquired spectrum. In this case, not only data for the spectrum in a predetermined interval but also all variables are averaged. The Interval averaging transformation may also improve the relationship between the concentration and spectrum of the measured spectrum by removing the noise peaks due to noise of the spectrometer system.

⑤ Transformation Condition 5: Interval removal Transformation

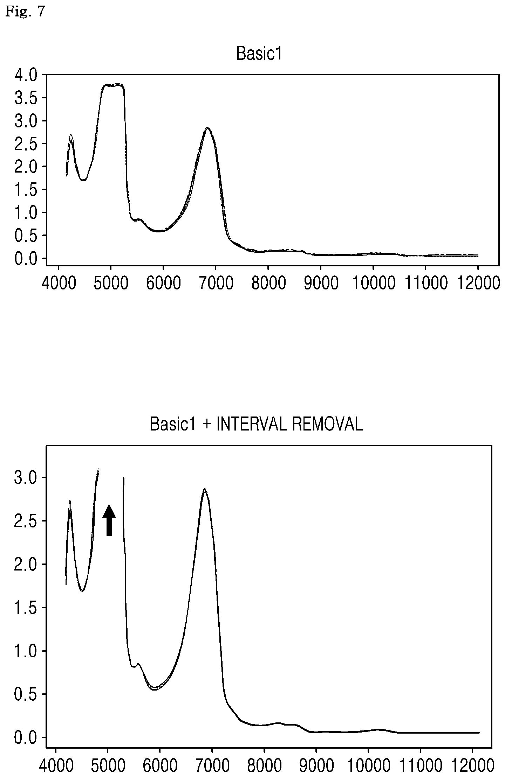

FIG. 7 shows a spectrum transformation method using an interval removal transformation method according to an embodiment of the present invention. Interval removal transformation refers to a transformation in which data values of a predetermined interval are removed in the acquired spectrum. When there are peaks or data that are not related to the analyte to be measured among the peaks or data of the spectrum, the relationship between the concentration and spectrum of the measured substance in all the intervals may be improved through the interval removal transformation.

(2) Derivation step for optimal transformation condition (Mo)

This is a step of deriving an optimal transformation condition through the procedure described below.

The inventors of the present invention have found that, when, in a function value of a reference similarity standard deviation Ak, a correlation coefficient Bk with a concentration, and a multivariate ratio Ck of the transformed spectra M1 S1, M2S2, ..., MkSi transformed under each transformation condition, a transformation condition under which the function value becomes maximum is set as the optimal transformation condition, the optimal prediction model is generated, and have applied it to the present invention.

In particular, a transformation condition under which a value of Fk (Ak, Bk, Ck) = aAk + bBk + cCk, which is an expression of a linear function of the Ak, Bk, and Ck values becomes maximum is set as the optimal transformation condition. Here, in the case of transformation under the transformation condition M1, the linear function is expressed as F1 (A1, B1, C1), and a, b, and c are constant values set by the user, and may be omitted.

That is, in the present invention, the optimal transformation condition means deriving k for which the Fk (Ak, Bk, Ck) is maximized, and the value of Fo (Ao, Bo, Co) = Ao + Ao + Co for the spectrum transformed under the optimal transformation condition Mo is the maximum value among Fk (k = 1, 2, ..., n) values.

Preferably, in the present invention, the optimal transformation condition means deriving k for which the Fk (Ak, Bk, Ck) is maximized, and the value of Fo (Ao, Bo, Co) = aAo + bAo + cCo for the spectra transformed under the optimal transformation condition Mo is the maximum value among the values of Fk (k = 1, 2, ..., n), where constant values of a, b, and c may be values selected from ranges of 6.5 to 7.7, 0.5 to 2.0, and 2.5 to 4.0, and more preferably, a, b, and c may be selected to be 6.69, 1.52, and 3.59, respectively.

For example, in the spectrum optimal transformation condition derivation unit of the present invention, for k transformed spectra obtained by transforming and combining each of the i spectra Si acquired as training data under the above-described transformation conditions 1 to 10, an Fk value is calculated, and a k-th transformation condition Mk showing the largest Fk is derived as the optimal transformation condition Mo.

Hereinafter, values of the reference similarity standard deviation Ak, the correlation coefficient Bk with the concentration, and the multivariate ratio Ck of transformed spectra are explained.

**① Reference similarity standard deviation (A)**

In order to derive the optimal spectrum transformation condition, a reference similarity standard deviation between each of the transformed spectra, which have been transformed according to the above spectrum transformation methods or a combination thereof and subjected to different transformation conditions, and a reference spectrum is calculated.

Here, the reference spectrum is a spectrum in the form of a straight line with the intensity of K (a non-zero constant) in all wavelength bands, and therefore, the reference similarity standard deviation is calculated as the reference similarity standard deviation between all transformed spectra and the reference spectrum. For example, when there are ten samples S1, S2, ..., S10 for the basic spectrum before transformation and each is transformed by ten transformation combination methods M1, M2, ..., M10, 100 transformed spectra M1S1, ..., M1S10, M2S1, ..., M2S10, ..., M10S10) are generated, and 100 reference similarities may be obtained based on the reference spectrum.

The reference similarity of the transformed spectra is obtained according to each of the above transformation conditions.

For example, the reference similarity standard deviation A1 is obtained for the transformed spectrum M1Si transformed according to the transformation condition M1, and in this way, the reference similarity standard deviation Ak for the transformed spectra M2Si, ..., MkSi transformed according to the transformation conditions M2, ..., Mk, ... is calculated.

The reference similarity standard deviation is obtained by calculating a cosine similarity of the transformed spectra transformed under a different transformation condition with respect to the reference spectrum, where the cosine similarity represents a similarity that is possible to obtain by using a straight-line reference spectrum and a transformed spectrum, that is, a cosine angle between the two spectra, and the closer the value is to 1, the higher the similarity may be determined. The reference similarity is calculated according to Equation 1 below.

[Equation 1]

$$Reference\ Similarity = \frac{\alpha \cdot \beta}{\|\alpha\|\|\beta\|} = \frac{\sum_{i=1}^{n} \alpha_i \times \beta_i}{\sqrt{\sum_{i=1}^{n}(\alpha_i)^2} \times \sqrt{\sum_{i=1}^{n}(\beta_i)^2}}$$

In the above Equation 1, $\alpha$ refers to the combination of the intensities of the combined transformed spectra, and $\beta$ refers to the combination of the intensities of the reference spectra. The intensity of the reference spectrum is an adjustable constant with a constant value for all multi variates.

Through Equation 1 above, reference similarity values between transformed spectra according to each of the transformation conditions and the reference spectrum are calculated, and the standard deviation of the reference similarities refers to the result value Ak of calculating the standard deviation between the reference similarities. In another embodiment, when the number of transformed spectra is more than a predetermined standard, the standard deviation of the reference similarities may refer to a result value calculated by dividing the standard deviation between similarities by the similarity average (standard deviation between reference similarities/similarity average). Therefore, the reference similarity standard deviation A calculated in the present invention is a value that allows the degree of difference between individual transformed spectra to be inferred, which makes it possible to sensitively recognize the difference in shape between transformed spectra.

The reference similarity standard deviation for the transformed spectrum $M_1S_i$ transformed under the transformation condition Mi is A1, and A1 is calculated as ( $\frac{\text{standard deviation between reference similarities}}{\text{similarity average}}$ ) of the transformed spectra $M_1S_1$, $M_1S_2$, ..., $M_1S_i$.

In this way, the reference similarity standard deviation $A_k$ for the transformed spectrum $M_kS_i$ transformed under the transformation condition $M_k$ is obtained.

### ② Correlation coefficient with substance concentration (B)

The relationship with the substance concentration refers to a normalized value B obtained by normalizing the covariance value representing the relation between the reference similarity of the spectrum and the corresponding actual substance concentration (actually measured concentration LCi) to a maximum value of 1. Since the value obtained by normalizing the covariance value to a maximum value of 1 is equal to a correlation coefficient of covariance characterized by having a range from -1 to +1, the normalized value, even if the correlation coefficient of covariance is calculated, has the same resulting value. That is, the calculation of the relationship with the target substance concentration makes it possible to estimate the relationship between the shape of the transformed spectrum and the corresponding substance concentration through B.

The covariance normalization value or the covariance correlation coefficient is calculated as in Equation 2 below, and the resulting values all have a range of -1 or more and +1 or less.

[Equation 2]

$$\rho = \frac{Cov(X,Y)}{\sqrt{Var(X)\,Var(Y)}} \quad , \quad -1 \le \rho \le 1$$

In Equation 2 above, X refers to the reference similarity, and Y indicates the actual substance concentration of the analyte.

In addition, Cov(X, Y) indicates a covariance value of the reference similarity X and a corresponding analyte concentration Y, and Var(X) and Var(Y) indicate variance values of the reference similarity X and the corresponding analyte concentration Y

In the above, the actual substance concentration refers to the actual concentration of a substance that is directly measured or known through an experiment or a separate method. This process is a process in which the relationship between actual substance concentration and the transformed spectrum is reflected as a training element.

In the above, LCi values, which are actual concentration values of substances corresponding to the spectra, which are acquired as training data, are input as Y values in Equation 2 above. As the X values, the reference similarity values calculated above are input.

The normalized value Bk of the covariance value calculated by the above calculation formula is calculated for the combination of the transformed spectra. For example, the reference similarity is obtained for the transformation combination method $M_k$ and the transformed spectrum M1Si, and accordingly, the normalized value Bk of the covariance value is calculated with the actual concentration value LCi of the corresponding substance.

③ **Multivariate ratio before and after spectrum transformation (C)**

In the present invention, a multivariate ratio C is calculated as another variable of the optimal spectrum transformation condition. The multivariate ratio is defined as C =

$$\frac{Multivariate\ number\ after\ spectrum\ tranformation}{Multivariate\ number\ before\ spectrum\ transformation}$$

. In the present invention, a multivariate number refers to the number of pieces of data acquired in a predetermined wavelength band corresponding to an X-axis of the acquired spectrum. For example, when spectra of a wavelength band of 1000 nm to 3000 nm are acquired by 1 nm in a spectrometer, the multivariate number at this time is (3000 - 1000), that is, 2000. If the spectra of 1000 to 1500 nm are removed through interval removal transformation among the transformation methods, the multivariate number after transformation is (3000 - 1500), meaning that the total number of multivariate variables is 1500. As another example, in the case of interval averaging transformation, the multivariate number is reduced by the set number of intervals.

In this way, it is possible to obtain the C value by calculating the multivariate number before the spectrum transformation versus the multivariate number after the spectrum transformation, and C at this time makes it possible to estimate degrees of freedom for the number of variables after spectrum transformation when spectrum transformation is learned by a machine learning (ML) model.

The multivariate ratio $C_k$ calculated by the above-described calculation formula varies depending on the number of pieces of data before and after the spectrum transformation, and thus represents a representative value of the degree of freedom for the number of pieces of data due to the spectrum transformation.

**(3) Calculation of optimal transformed spectrum (MoSi) data**

After obtaining the transformation condition Mo that maximizes $F_k$ ($A_k$, $B_k$, $C_k$), for i * k transformed spectra $M_kSi$ in the above-described method, the basic spectrum Si is transformed under the transformation condition Mo to calculate the optimal transformed spectrum (MoSi) data. As the optimal transformed spectrum (SoSi) data, the transformed spectrum (MoSi) data having k=o generated in the $F_k$ ($A_k$, $B_k$, $C_k$) calculation process may be used as it is.

**1.2 Generation step for machine learning and concentration prediction model (S40)**

In the present invention, the concentration prediction model is generated by performing machine learning using the optimal transformed spectra MoSi obtained by transforming the above-described basic spectra Si with the optimal transformation condition Mo through machine learning and the actually measured concentration $LC_i$ of respective corresponding substances as training data.

A neural network or learning model used for training the concentration prediction model generated in the present invention is not limited to one specific method, and a neural network or learning model that generates a concentration prediction model from known spectrum data may be used.

The concentration prediction model generated by performing learning with the optimal transformed spectrum MoSi in this way is used for concentration prediction in the concentration prediction step to be described later.

**1.3 Concentration prediction step**

The concentration of a new substance is predicted by using the generated concentration prediction model. The concentration prediction includes a prediction spectrum (PS) acquisition step, an optimal transformation prediction spectrum generation step of generating an optimal transformation prediction spectrum P.MoS by transforming the acquired prediction spectrum under the above-described optimal transformation condition, and a prediction concentration output step of outputting a concentration prediction value PLC by inputting the optimal transformation prediction spectrum (PMoS) data to the generated concentration prediction model.

**(1) Prediction spectrum (PS) acquisition step (P10)**

This step is a step of acquiring a spectrum of a substance to be predicted in concentration through a spectrum acquisition unit.

**(2) Optimal transformation prediction spectrum (P.MoS) generation step (P20)**

This is a step of generating an optimal transformation prediction spectrum by transforming a prediction spectrum under the above-described optimal transformation condition.

**(3) Prediction concentration (PLC) output step (P30)**

This is a step of inputting the above-described optimal transformation prediction spectrum into the concentration prediction model generated by performing machine learning with a set of the above-described optimal transformed spectra, and acquiring a concentration prediction value (PLC) as an output thereof.

**<Performance validation of the prediction model based on the detection of the optimal transformation conditions of the present invention>**

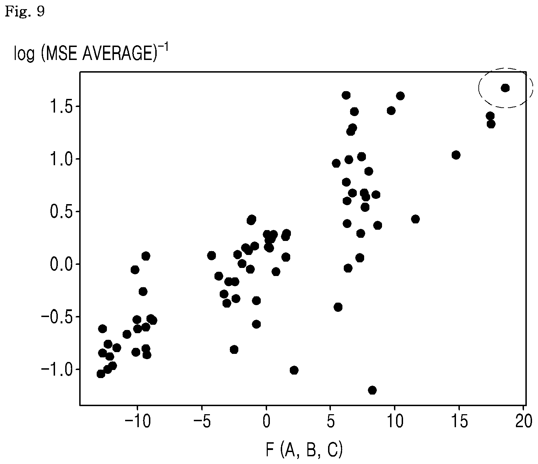

FIG. 9 shows mean squared error according to spectrum transformation conditions. A graph shows $\log(\text{MSE average})^{-1}$ according to F (A,B,C), where MSE refers to mean squared error, and in FIG. 9, it can be seen that the higher F (A, B, C), the higher the $\log(\text{MSE average})^{-1}$. The graph indicates that the lower the mean square error, the smaller the difference in concentration prediction values through the ML model, and thus the higher the accuracy of the generated ML model. That is, the lower the MSE average, the more advantageous it is to generate an ML model with high accuracy through spectrum transformation.

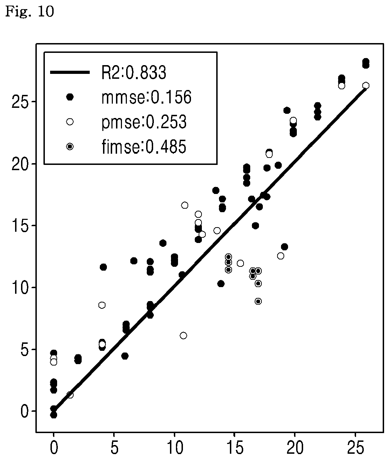

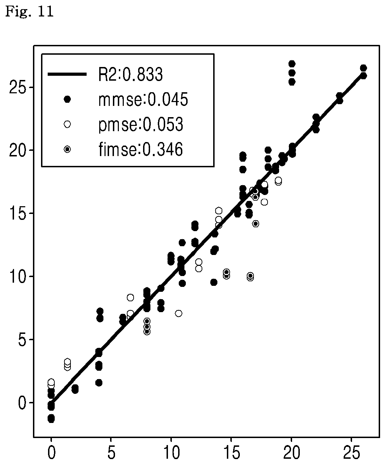

FIGS. 10 and 11 are graphs showing a concentration prediction result of the concentration prediction method in the related art and a concentration prediction result of the concentration prediction method of the present invention, respectively. FIG. 10 shows a verification result for a model performing DNN learning without spectrum transformation according to the related art, and FIG. 11 shows a validation result for a model performing DNN learning with optimal spectrum transformation according to the present invention.
In the generation and validation of the learning model in the present invention, the data used were predicted and verified with a model that predicts the concentration of glucose used as a substrate from the real-time spectrum of the process solution in the incubator. In this validation, the value of Fo (Ao, Bo, Co) = aAo + bAo + cCo for the spectra transformed under the optimal transformation condition Mo is the maximum value among the Fk (k = 1, 2, ..., n) values, and at this time, a, b, and c were applied as 6.69, 1.52, and 3.59, respectively.
A line represented by $R^2$ represents a coefficient of regression determination, which means that the larger the value, the better the prediction result of the model.
In order to evaluate the trained and generated concentration prediction ML model, some data is excluded in advance so that it is not used for training and generating of the concentration prediction ML model, and the performance of the model is tested using the excluded data, and such data is referred to as validation data. fimse marked with green dots in the above graphs represents the result of evaluating the mean squared error based on the validation data not used for training and generating the ML model. In addition, in the graphs above, mmse marked with black dots represents the result of evaluating the mean squared error (MSE) based on the training data used to generate the ML model as the result of training and generating the ML model, and pmse marked with red dots represents the result of evaluating the mean squared error based on input data not used for training and generating the ML model.
Looking at the concentration prediction accuracy of the ML model generated through the optimal spectrum transformation process of the present invention in FIG. 11, it can be seen that the $R^2$ value is higher than that of the related art of FIG. 10, and it can be also seen that pmse and fimse point data are also more precisely and accurately distributed on the prediction validation line.

**2. Substance concentration prediction system 100 according to present invention**

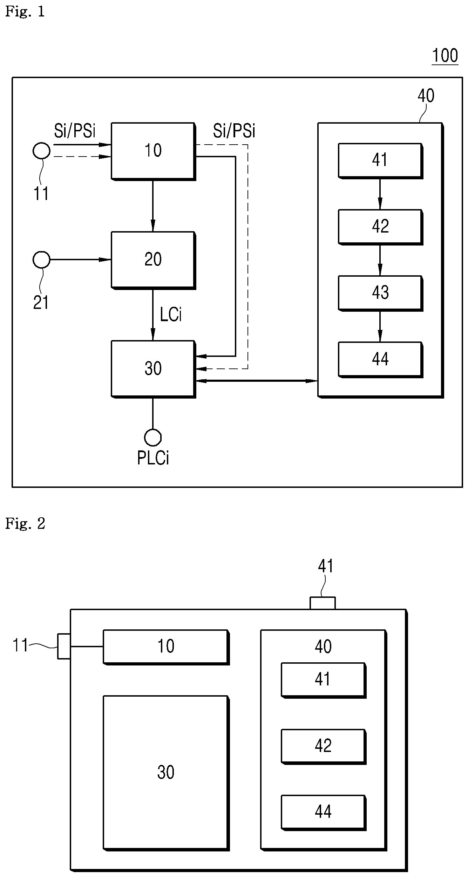

FIG. 1 is a diagram showing a substance concentration prediction system 100 according to the present invention.
Referring to FIG. 1, the present invention is based on a spectrometer for an optical analysis method, where, using a spectrometer, spectra of a substance of a known concentration and a substance to be predicted in concentration are acquired, and the acquired spectra are analyzed to output the concentration value of the substance. In addition, the detected spectrum and the concentration value of the analyte are applied as training data for the concentration prediction model, the optimal transformation condition of the spectrum for more accurate concentration prediction is derived from the concentration prediction model, and the concentration prediction model predicts the concentration of the analyte to be predicted by transforming the spectra under the optimal transformation condition.
In FIG. 1, the flow of data indicated by a solid line indicates the flow of data used to generate the concentration prediction model, and the flow of data indicated by a dotted line indicates the flow of data according to the procedure of predicting the concentration of the substance using the generated concentration prediction model.
The substance concentration prediction system 100 of the present invention includes a spectrum data acquisition unit 10, a concentration data acquisition unit 20, a calculation and control device 30, and a memory device 40. Each configuration is described below.

### 2.1. Spectrum data acquisition unit 10

As the spectrum acquisition unit, a known spectrometer and spectrophotometer may be used for optical analysis. A spectrum of an analyte to be measured in concentration is acquired by using the spectrometer, and the acquired spectrum may be calculated as a concentration value of the analyte by using an analysis method according to the type of spectrometer.

The spectrum acquisition unit of the present invention acquires the basic spectrum Si for training data and a prediction spectrum Psi for concentration prediction. The spectrum acquisition unit 10 may acquire the basic spectrum or prediction spectrum from an external device such as the spectrometer, an external spectrum generating device, a memory 40, or the like, and may be provided with a data connection unit 11 for data connection.

(1) Acquisition of basic spectrum $S_i$

The spectrum data acquisition unit 10 acquires basic spectra of substances of known concentrations from the spectrometer, and uses the acquired basic spectrum set as training data. In the present invention, the acquired i spectra S are expressed as $S_1$, $S_2$, .... $S_i$. The basic spectra become the basis for generating a transformed spectrum MSi to be used as training data in a machine learning module 43 to be described later.

The basic spectrum Si may be pre-stored in the memory in the form of previously acquired spectrum data, and in this case, the spectrum acquisition unit acquires the basic spectrum Si from the memory.

(2) Acquisition of prediction spectrum PSi

The spectrum data acquisition unit 10 also acquires the prediction spectrum PSi of the substance to be predicted in concentration, which is be used as an input of the generated concentration prediction model. The acquired prediction spectrum data is transformed to a predicted optimal transformed spectrum PMoSi according to the optimal transformation condition, and is input to the concentration prediction model generated according to the above-described procedure to be used to predict the concentration of the substance.

### 2.2. Substance concentration data (LCi) acquisition unit 20

This is a configuration of acquiring the concentration data LCi for the substance corresponding to each of the above-described basic spectra. In the case of a substance of a known concentration, the concentration data LCi may be stored in the memory and acquired by reading the memory by the substance concentration data (LCi) acquisition unit 20; in contrast, the concentration data LCi of the substance corresponding to the basic spectrum is acquired by using a known concentration measuring device (not shown) linked to the concentration measuring system of the present invention or by performing measurement experimentally.

The concentration data acquisition unit 20 may acquire known or experimentally measured concentration data from an external concentration measuring device, an external experimental data storage, the memory 40, or the like, and may be provided with a data connection unit 21 for data connection.

### 2.3. Calculation and control device 30

The calculation and control device 30 of the present invention may be a single CPU or microprocessor, or a terminal device, computer device, computer server, or the like including the CPU or microprocessor.

The calculation and control device 30 controls the overall operation of the system, and acquires and stores data, performs machine learning, generates the concentration prediction model, and manages the concentration prediction model generated as a result of machine learning.

In addition, the calculation and control device 30 oversees the transformation of the spectrum of the present invention, derivation of the optimal transformation condition through the transformation, generation of transformed spectra according to the optimal transformation condition, training of the concentration prediction model using the generated transformed spectra, and generation of the concentration prediction model.

In performing the above operations, the calculation and control device of the present invention may utilize computer programs for controlling respective functions loaded in the memory, store the generated concentration prediction model in the memory, read the concentration prediction model back from the memory when the concentration is predicted, and perform the prediction calculation. The computer programs that perform respective functions are stored in a memory device to be described later as each of divided modules, and it is easily understood by a person skilled in the art that the calculation and control devices may perform the functions of each module by reading the computer programs from the memory when necessary.

Hereinafter, each operation performed by the calculation and control device of the present invention will be described.

**(1) Spectrum transformation and combination**

The transformed spectra MkSi are generated by transforming and combining the basic spectra Si acquired by the spectrum acquisition unit. The operation is the same as described in Section 1.1.3 above. In performing this procedure, the calculation and control device may read and perform a spectrum transformation and combination module or transformation algorithm stored in the form of software/programs in the memory.

In the present invention, for the spectrum transformation and combination procedure, a separate spectrum transformation and combination unit may be configured to perform the spectrum transformation and combination procedure according to the spectrum transformation algorithm, such as a microprocessor or the like provided as a separate module in the calculation and control device.

**(2) Derivation of optimal transformed spectrum**

The optimal transformation condition is derived from the transformed spectra MkSi described above. The specific procedure for deriving the optimal transformation condition is the same as that described in Section 1.1.3 above, and may be performed by reading an optimal spectrum derivation module loaded in the form of software/programs into the memory, which will be described later.

① The optimal transformed spectrum used as training data to generate the concentration prediction model is calculated by obtaining the optimal transformation condition from the transformed spectra for the basic spectra Si and transforming the basic spectra Si with the optimal transformation condition, or among previously generated transformed spectra, a transformed spectrum that meets the optimal transformation condition is selected as the optimal transformed spectrum. The derived transformed spectrum is used as input data for machine learning to be described later.

The derived optimal transformation condition Mo may be stored in the memory for later use.

② In the case of trying to predict the concentration, the calculation and control device transforms the prediction spectrum PSi of the target substance with the previously calculated optimal transformation condition Mo to generate the optimal transformation prediction spectrum MoPSi. The optimal transformation prediction spectrum MoPSi is input to a concentration prediction module 44 to calculate the concentration prediction value PLC.

In another embodiment, the present invention may be configured such that the spectrum transformation and combining unit is separately provided such as a separate microprocessor or the like to perform an optimal transformed spectrum derivation procedure.

**(3) Machine learning and generation of concentration prediction model**

The calculation and control unit of the present invention generates the concentration prediction model as described in Section 1.2 above by performing machine learning using the optimal transformed spectra MoSi obtained by transforming the above-described basic spectra Si with the optimal transformation condition Mo through machine learning and the actually measured concentration (LCi) of respective corresponding substances as training data. The generated concentration prediction model may be stored in the memory in the form of a software program or algorithm.

**(4) Prediction of concentration**

The concentration prediction value PLC is calculated by inputting the previously generated optimal transformation prediction spectrum MoPSi into the generated concentration prediction model.

**2.4. Memory device 40**

In the concentration prediction system of the present invention, the memory device 40 may be used as a data storage for pre-storing concentration value data LCi and basic spectra Si of substances of known concentrations to be used as training data, and is loaded with each of the following computer software modules in the form of a computer program/software to perform respective functions of the system of the present invention. In addition, the memory device 40 stores the optimal transformation condition Mo calculated by the calculation and control device.

Meanwhile, the concentration prediction system of the present invention may be configured by being loaded with each of the following software modules, but may also be configured by separating each module into a separate storage. The memory device 40 mentioned in the present invention is not necessarily limited to one physical storage.

In addition, the memory of the present invention may be replaced with a memory device such as a computer system or a server system, and may be constructed as a movable memory device including a separate CD, hard disk, USB, or the like, including each program module or each software algorithm described below.

Each 'module' described below may refer to a series of computer software algorithms configured to perform a corresponding function.

### (1) Spectrum transformation and combination module 41

The memory of the present invention is loaded with a computer software module or transformation algorithm that performs the transformation method performing (1) spectrum transformation and combination step, which has been described in the above acquisition step for the optimal transformed spectrum of Section 1.1.3, and performs the transformation.

### (2) Optimal spectrum derivation module 42

The memory of the present invention is loaded with a computer software module that performs (2) derivation step for the optimal transformation condition, which has been described in the above acquisition step for the optimal transformed spectrum of Section 1.1.3, and calls the spectrum transformation and combination module to calculate the optimal transformed spectrum according to the optimal transformation condition.

### (3) Machine Learning Module 43

The memory of the present invention is loaded with a machine learning module, which is a computer software module that (3) calculates optimal transformed spectrum data, which has been described in the above acquisition step for the optimal transformed spectrum of Section 1.1.3, performs the machine learning process described above in Section 1.2 by using the calculated data as training data together with the concentration data $LC_i$ for the substances of the known concentrations to generate the concentration prediction model.

### (4) Concentration prediction module 44

A computer software module that performs the concentration prediction model generated by the machine learning module 43 described above is loaded.

In trying to predict the concentration of a predetermined substance, the calculation and control device 30 uses the prediction spectrum $PS_i$ of the substance to be predicted in concentration as input data to input the optimal transformation prediction spectrum $PMoS_i$, which is the transformed spectrum of the prediction spectrum $PS_i$, into the concentration prediction module 44 of a prediction memory, and the concentration prediction module 44 calculates a concentration prediction value $PLC_i$.

The concentration measuring system of the present invention may be configured to change and set the optimal transformation condition to correspond to the different optimal transformation condition being set differently depending on the type of substance to be measured. The calculation and control device 30 accepts a selection input displayed on a screen display unit (not shown) of the system or a selection input input from a physical switch (not shown) to receive a predetermined optimal transformation condition that has been determined, calls the corresponding spectrum transformation module from the memory 40 and transforms the spectrum, and inputs the transformed spectrum back into the concentration prediction model called from the memory 40 and performs the calculation to output the prediction concentration value. In this case, it will be apparent to those skilled in the art that the concentration prediction modules 44 corresponding to a plurality of concentration prediction models corresponding to respective specific optimal transformation conditions may be generated according to the method for generating the concentration prediction model, which has been described above, and the concentration prediction modules 44 may be stored in the memory 40.

### 3. Concentration measuring device according to present invention

Referring to FIG. 2, a concentration measuring device loaded with a concentration prediction module 44 generated according to the technical idea of the present invention will be described.

The concentration measuring device of the present invention is equipped with the spectrum data acquisition unit 10, the calculation and control device 30, and the memory device 40 described in the previous Section 2. Each configuration may be set differently from the concentration measuring system described in the previous Section 2, which is described in detail below.

### (1) Spectrum data acquisition unit 10

The spectrum data acquisition unit 10 of the concentration measuring device of the present invention acquires prediction spectrum (PSi) data for a target substance. As input, data output by well-known spectrometers, spectrophotometers, optical sensors, or the like, that output spectrum data for a substance to be measured by contact or non-contact in concentration is included.

### (2) Memory device 40

The memory device 40 of the concentration measuring device of the present invention is loaded with (1) the spectrum transformation and combination module loaded in the memory device 40 described in Section 2.4 and (4) the concentration prediction module 44 according to the derived optimal transformation condition and the previously learned optimal transformed spectrum.

The memory device 40 of the concentration measuring device may be configured to, depending on the purpose, include only the spectrum transformation module according to the optimal transformation condition previously calculated among the spectrum transformation and combination modules, and in this case, the concentration prediction module 44 to be loaded is also the concentration prediction module 44 that has performed learning in advance using the optimal transformed spectrum as training data.

### (3) Calculation and control device 30

The calculation and control device 30 of the concentration measuring device of the present invention utilizes the optimal transformation condition, spectrum transformation and combination module, and concentration prediction module 44 loaded in the memory device 40 to calculate the concentration prediction value PLCi from the prediction spectrum (PSi) data for the acquired target substance.

The concentration measuring device of the present invention is configured to change and set the optimal transformation condition to correspond to the optimal transformation condition set differently depending on the type of substance to be measured. The calculation and control device 30 accepts a selection input displayed on a screen display unit (not shown) or a selection input input from a physical switch (not shown) to receive a predetermined optimal transformation condition that has been determined in advance, calls the corresponding spectrum transformation module from the memory device 40 and transforms the spectrum, and inputs the transformed spectrum back into the concentration prediction model called from the memory device 40 and performs the calculation to output the prediction concentration value. In this case, it will be apparent to those skilled in the art that the concentration prediction modules 44 corresponding to a plurality of concentration prediction models corresponding to respective specific optimal transformation conditions may be generated according to the method described above and the concentration prediction modules 44 may be stored in the memory 40, and a control may be performed to calculate the prediction concentration by calling the concentration prediction module 44 matching with the corresponding substance. Meanwhile, the concentration prediction module 44 may not be generated by learning a specific transformed spectrum, but a general-purpose spectral concentration prediction model may be used.

### (4) Data connection unit 21

Meanwhile, the concentration measuring device of the present invention may further include a data connection unit for connecting an external memory device or inputting and receiving data into and from an external device.

The data connection unit may be integrated with the spectrum data acquisition unit 10 to receive spectrum data and other data from an external device.

The spectrum transformation and combination module 41, the optimal transformation conditions, and the previously generated concentration prediction model may be received and stored in the memory device 40 through the data connection unit, and through the configuration, the optimal transformation conditions, the spectrum transformation and combination module 41, and the concentration prediction model, which are set differently according to the nature of the substance to be measured in concentration, are stored in the memory device 40 of the concentration measuring device to be applied to the concentration measurement.

The data connection unit may be configured to connect an external movable memory device 40, and in this case, the configuration is established such that the spectrum transformation and combination module 41, the optimal transformation condition, and the previously generated concentration prediction model are stored in an external memory device connected additionally to or in place of the memory device 40, and the calculation and control device 30 reads them so as to utilize the concentration prediction.

Names of the reference signs used in the specification and drawings of the present invention are as follows.

> 100: Concentration prediction system
> 10: Spectrum data acquisition unit
> 20: Substance concentration data acquisition unit
> 30: Calculation and control device
> 40: Memory device
> 41: Spectrum transformation and combination module
> 42: Optimal spectrum derivation module
> 43: Machine learning module
> 44: Concentration prediction module

**Claims**

1. A method for generating a concentration prediction model for predicting a concentration of a target substance through machine learning, the method comprising:

   generating a transformed spectrum by transforming a base spectrum of a substance of a known concentration according to a predetermined transformation condition; and
   generating a concentration prediction model by inputting the transformed spectrum and a measured concentration of a substance corresponding to the transformed spectrum into a machine learning algorithm.

2. The method of claim 1, wherein the predetermined transformation condition is derived from:
   transformed spectra generated by transforming a spectrum with two or more different transformation conditions.

3. The method of claim 2, wherein deriving the transformation condition comprises calculating each of a reference similarity standard deviation of the transformed spectra, a correlation coefficient with the measured concentration, and a multivariate ratio before and after spectrum transformation, and wherein the transformation condition is that a sum of the reference similarity standard deviation, the correlation coefficient and the multivariate ratio is maximum.

4. The method of claim 2, wherein deriving the transformation condition comprises calculating each of a reference similarity standard deviation Ak that is a standard deviation between reference similarities of the transformed spectra for each transformation condition, a correlation coefficient Bk with the measured concentration, and a multivariate ratio Ck before and after spectrum transformation, and calculating constant values a, b, and c for which a value of a linear function Fk (Ak, Bk, Ck) = aAk + bBk + cCkis maximum, and
   wherein the reference similarity for obtaining the reference similarity standard deviation, the correlation coefficient with the actually measured concentration, and the multivariate ratio before and after spectrum transformation are calculated by Equations 1, 2, and 3 below, respectively,

Equation 1)

$$\text{Reference Similarity} = \frac{\alpha \cdot \beta}{\|\alpha\| \|\beta\|} = \frac{\sum_{i=1}^{n} \alpha_i \times \beta_i}{\sqrt{\sum_{i=1}^{n}(\alpha_i)^2} \times \sqrt{\sum_{i=1}^{n}(\beta_i)^2}}$$

wherein $\alpha$ is a combination of intensities of combined transformed spectra, and $\beta$ is a combination of intensities of the reference spectrum,

Equation 2)

$$\rho = \frac{Cov(X,Y)}{\sqrt{Var(X)\,Var(Y)}}, \qquad -1 \le \rho \le 1$$

wherein X is the reference similarity, Y is a substance concentration of an analyte, Cov(X, Y) is a covariance value of the reference similarity (X) and the corresponding substance concentration (Y), and Var(X) and Var(Y) are variance values of the reference similarity (X) and the corresponding substance concentration (Y), and

Equation 3)

$$C = \frac{Multivariate\ number\ after\ spectrum\ tranformation}{Multivariate\ number\ before\ spectrum\ transformation}$$

wherein multivariate number is a total number of pieces of data acquired within a predetermined wavelength band corresponding to an X-axis of an acquired spectrum.

5. A non-transitory computer-readable medium having a concentration prediction model generation algorithm programmed thereon, the concentration prediction model generation algorithm comprising:

a spectrum transformation module for generating transformed spectra corresponding to base spectra by transforming the base spectra according to two or more transformation conditions;
a transformed spectrum calculation module for calculating each of a reference similarity standard deviation of each of the transformed spectra according to the transformation conditions, a correlation coefficient with a measured concentration of a substance corresponding to each of the transformed spectra, and a multivariate ratio before and after spectrum transformation, and calculating the transformation spectra according to a transformation condition for which a sum of the reference similarity standard deviation, the correlation coefficient and the multivariate ratio is maximum; and
a machine learning algorithm generation module for generating a concentration prediction model using measured concentrations of substances corresponding to the transformed spectra as training data.

6. A concentration prediction method for predicting a concentration of an analyte, the concentration prediction method comprising:

acquiring a prediction spectrum of a substance to be predicted in concentration; generating a transformation prediction spectrum by transforming the acquired prediction spectrum under a transformation condition; and outputting a concentration prediction value by inputting the transformation prediction spectrum to a concentration prediction model,
wherein the optimal transformation condition is a transformation condition derived from:

generating transformed spectra by transforming base spectra of predetermined substances having concentration values known by measurement with two or more different transformation conditions; and deriving the transformation condition from the generated transformed spectra,

wherein the concentration prediction model is a neural network or a concentration prediction algorithm trained to calculate a predicted concentration value from transformed spectra obtained by transforming the base spectra with the transformation condition by inputting the transformed spectra and measured concentration values corresponding to the transformed spectra into a machine learning algorithm.

7. The concentration prediction method of claim 6, wherein deriving the transformation condition comprises calculating each of a reference similarity standard deviation of the transformed spectra, a correlation coefficient with the measured concentration, and a multivariate ratio before and after spectrum transformation, and wherein the transformation condition is that a sum of the reference similarity standard deviation, the correlation coefficient and the multivariate ratio is maximum.

8. The concentration prediction method of claim 6, wherein deriving the transformation condition comprises calculating each of a reference similarity standard deviation $A_k$ that is a standard deviation between reference similarities of the transformed spectra for each transformation condition, a correlation coefficient $B_k$ with the measured concentration, and a multivariate ratio $C_k$ before and after spectrum transformation, and calculating constant values a, b, and c for which a value of a linear function $F_k$ ($A_k$, $B_k$, $C_k$) = $aA_k$ + $bB_k$ + $cC_k$ is maximum, and wherein the reference similarity for obtaining the reference similarity standard deviation, the correlation coefficient with the actually measured concentration, and the multivariate ratio before and after spectrum transformation are calculated by Equations 1, 2, and 3 below, respectively,

Equation 1)

$$\text{Reference Similarity} = \frac{\alpha \cdot \beta}{\|\alpha\|\|\beta\|} = \frac{\sum_{i=1}^{n} \alpha_i \times \beta_i}{\sqrt{\sum_{i=1}^{n}(\alpha_i)^2} \times \sqrt{\sum_{i=1}^{n}(\beta_i)^2}}$$

wherein $\alpha$ is a combination of intensities of combined transformed spectra, and $\beta$ is a combination of intensities of the reference spectrum,

Equation 2)

$$\rho = \frac{Cov(X,Y)}{\sqrt{Var(X)\,Var(Y)}} \,, \ -1 \leq \rho \leq 1$$

wherein X is the reference similarity, Y is a substance concentration of an analyte, Cov(X, Y) is a covariance value of the reference similarity (X) and the corresponding substance concentration (Y), and Var(X) and Var(Y) are variance values of the reference similarity (X) and the corresponding substance concentration (Y), and

Equation 3)

$$C = \frac{Multivariate\ number\ after\ spectrum\ tranformation}{Multivariate\ number\ before\ spectrum\ transformation}$$

wherein multivariate number is a total number of pieces of data acquired within a predetermined wavelength band corresponding to an X-axis of an acquired spectrum.

9. A concentration prediction system for predicting a concentration of an analyte, the concentration prediction system comprising:

a spectrum data acquisition unit configured to acquire a base spectrum of a substance for generating training data or a prediction spectrum that is a spectrum of a substance to be measured in concentration;
a substance concentration data acquisition unit configured to acquire a measured concentration of the substance to be measured through previously acquired concentration data for the substance and a concentration measuring device;
a calculation and control device configured to generate transformed spectra of the base spectrum and the prediction spectrum, calculate a transformation condition, perform machine learning, and generate a concentration prediction model; and
a memory for storing the base spectra of analytes acquired by the spectrum data acquisition unit, measured substance concentration values acquired by the substance concentration data acquisition unit, and the concentration prediction model generated by the calculation and control device.

10. The concentration prediction system of claim 9, wherein the calculation and control device is configured to:

perform a spectrum transformation and combination of spectra acquired in the spectrum data acquisition unit according to a spectrum transformation algorithm,
derive a transformed spectrum exhibiting concentration prediction accuracy as a transformed spectrum, among the transformed spectra,
generate a concentration prediction model by performing machine learning using the transformed spectrum and the measured concentration of a corresponding substance as training data, and
perform concentration prediction by inputting the prediction spectrum into the generated concentration prediction model.

11. The concentration prediction system of claim 9, wherein the memory has stored thereon:

a spectrum transformation and combination module for transforming the spectrum acquired by the spectrum acquisition unit according to the transformation condition;

a spectrum derivation module for acquiring a spectrum exhibiting concentration prediction accuracy among the transformed spectra and deriving the transformation condition;

a machine learning module for performing machine learning and generating a concentration prediction model using the transformed spectrum and measured substance concentration as training data; and

a concentration prediction module for calculating a predicted concentration value by using the prediction spectrum as input data.

12. A non-transitory computer-readable medium having a concentration prediction algorithm programmed thereon, the concentration prediction algorithm comprising:

a spectrum transformation module for generating a transformed spectrum by transforming a prediction spectrum of a substance to be predicted in concentration;

a spectrum derivation module for calculating a transformation prediction spectrum by calling the spectrum transformation module and transforming the prediction spectrum according to a transformation condition; and

a concentration prediction module for receiving the transformation prediction spectrum and calculating a predicted concentration value,

wherein the concentration prediction module is generated by performing machine learning based on a measured concentration of a substance corresponding to the spectrum transformed according to the transformation condition.

13. A concentration measuring device for predicting a concentration of an analyte, the concentration measuring device comprising:

a spectrum data acquisition unit configured to acquire a prediction spectrum that is a spectrum of an analyte;

a memory device having programmed thereon a spectrum transformation module for transforming a prediction spectrum according to a transformation condition and a concentration prediction module for calculating a concentration prediction value from a transformation prediction spectrum obtained by transforming the prediction spectrum according to the transformation condition; and

a calculation and control device configured to perform a control to read the concentration prediction module loaded in the memory device and calculate a concentration prediction value from a prediction spectrum to be predicted in concentration.

14. The concentration measuring device of claim 13, further comprising a data connection unit configured to connect an external memory device or receive data from the external memory device,

wherein the memory device is connected to the data connection unit in a detachable manner.

15. The concentration measuring device of claim 13, further comprising a data connection unit configured to connect an external memory device or receive data from the external memory device,

wherein the memory device is configured to receive and store a spectrum transformation module and a concentration prediction module from the external memory device through the data connection unit, wherein the spectrum transformation module is further for transforming the prediction spectrum according to a transformation condition.

16. The concentration measuring device of claim 14, wherein the data connection unit is integrated with the spectrum data acquisition unit.

Fig. 1

Fig. 2

Fig 3.

Basic1

Basic1 + inverse

Fig. 4

## Basic2(RANDOM INTERVAL)

## Basic2+filter

Fig. 5

Basic1

Basic1+ baseline removal

Fig. 6

## Basic2+filter

## Basic2+INTERVAL AVERAGING

Fig. 7

Basic1

Basic1 + INTERVAL REMOVAL

EP 4 400 828 A1

Fig. 8

Fig. 9

log (MSE AVERAGE)$^{-1}$

F (A, B, C)

Fig. 10

Fig. 11

## INTERNATIONAL SEARCH REPORT

| International application No. |
|---|
| **PCT/KR2022/018141** |

**A.    CLASSIFICATION OF SUBJECT MATTER**

**G01N 21/25**(2006.01)i; **G06N 20/00**(2019.01)i

According to International Patent Classification (IPC) or to both national classification and IPC

**B.    FIELDS SEARCHED**

Minimum documentation searched (classification system followed by classification symbols)

G01N 21/25(2006.01); A61B 5/00(2006.01); G01N 30/86(2006.01); G01N 30/88(2006.01); G06K 9/00(2006.01); G06K 9/62(2006.01); G06N 3/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 농도(density), 학습(learning), 모델(model), 스펙트럼(spectrum) 및 예측(prediction )

**C.    DOCUMENTS CONSIDERED TO BE RELEVANT**

| Category* | Citation of document, with indication, where appropriate, of the relevant passages | Relevant to claim No. |
|---|---|---|
| X | JP 2020-106340 A (CANON INC.) 09 July 2020 (2020-07-09)<br>See paragraphs [0017]-[0066] and figures 1-4. | 1 |
| Y | | 2-16 |
| Y | KR 10-2022-0046167 A (SAMSUNG ELECTRONICS CO., LTD.) 14 April 2022 (2022-04-14)<br>See paragraphs [0017] and [0046]-[0075] and figures 1-4g. | 2-16 |
| A | KR 10-2017-0057083 A (SAMSUNG ELECTRONICS CO., LTD.) 24 May 2017 (2017-05-24)<br>See paragraphs [0027]-[0100] and figures 1-11. | 1-16 |
| A | KR 10-2020-0099816 A (DAEGU GYEONGBUK INSTITUTE OF SCIENCE AND TECHNOLOGY) 25 August 2020 (2020-08-25)<br>See paragraphs [0030]-[0101] and figures 1-12. | 1-16 |

| ☑ Further documents are listed in the continuation of Box C. | ☑ See patent family annex. |
|---|---|

| \* | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **26 July 2023** | **26 July 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
|---|
| **PCT/KR2022/018141** |

**C.**     **DOCUMENTS CONSIDERED TO BE RELEVANT**

| Category* | Citation of document, with indication, where appropriate, of the relevant passages | Relevant to claim No. |
|---|---|---|
| A | CN 112395940 A (HAINAN UNIVERSITY et al.) 23 February 2021 (2021-02-23)<br>See paragraphs [0014]-[0033] and figure 1. | 1-16 |
| E | KR 10-2023-0011110 A (LG CHEM, LTD.) 20 January 2023 (2023-01-20)<br>See paragraphs [0033]-[0184]; claims 1-16; and figures 1-11. | 1-16 |

Form PCT/ISA/210 (second sheet) (July 2022)

**INTERNATIONAL SEARCH REPORT**
Information on patent family members

| International application No. |
| --- |
| **PCT/KR2022/018141** |

| Patent document cited in search report | | | Publication date (day/month/year) | Patent family member(s) | | | Publication date (day/month/year) |
| --- | --- | --- | --- | --- | --- | --- | --- |
| JP | 2020-106340 | A | 09 July 2020 | None | | | |
| KR | 10-2022-0046167 | A | 14 April 2022 | US | 2022-0104776 | A1 | 07 April 2022 |
| KR | 10-2017-0057083 | A | 24 May 2017 | KR | 10-2487983 | B1 | 11 January 2023 |
| | | | | US | 10729383 | B2 | 04 August 2020 |
| | | | | US | 11684317 | B2 | 27 June 2023 |
| | | | | US | 2017-0135645 | A1 | 18 May 2017 |
| | | | | US | 2020-0330048 | A1 | 22 October 2020 |
| KR | 10-2020-0099816 | A | 25 August 2020 | KR | 10-2527149 | B1 | 28 April 2023 |
| CN | 112395940 | A | 23 February 2021 | None | | | |
| KR | 10-2023-0011110 | A | 20 January 2023 | None | | | |

Form PCT/ISA/210 (patent family annex) (July 2022)

**REFERENCES CITED IN THE DESCRIPTION**

**Patent documents cited in the description**

- KR 20200018177 A

- JP 6871195 B